Europäisches Patentamt

European Patent Office

Office européen des brevets

(19)

(11)    EP 0 886 529 B1

(12)     **FASCICULE DE BREVET EUROPEEN**

(45) Date de publication et mention
de la délivrance du brevet:
**15.12.2004   Bulletin 2004/51**

(51) Int Cl.$^7$: **A61M 1/36**

(86) Numéro de dépôt international:
**PCT/IB1997/001289**

(21) Numéro de dépôt: **97943094.9**

(22) Date de dépôt: **17.10.1997**

(87) Numéro de publication internationale:
**WO 1998/017334 (30.04.1998 Gazette 1998/17)**

(54) **SYSTEME DE DETERMINATION DE LA RECIRCULATION DU SANG DANS UN ACCES VASCULAIRE**

VORRICHTUNG ZUR BESTIMMUNG DER BLUTREZIRKULATION IN EINEm VASKULäREN ZUGANG

DEVICE FOR DETERMINING THE RECIRCULATION OF BLOOD IN A VASCULAR ACCESS

(84) Etats contractants désignés:
**AT BE CH DE ES FR GB IT LI NL SE**

(30) Priorité:  **18.10.1996  IT  TO960855**

(43) Date de publication de la demande:
**30.12.1998   Bulletin 1998/53**

(60) Demande divisionnaire:
**04012686.4 / 1 449 549**

(73) Titulaire: **GAMBRO HOSPAL (Schweiz) AG**
**4001 Basel (CH)**

(72) Inventeurs:
• **CAVICCHIOLI, Giovanni**
**I-41036 Medolla (IT)**

• **CANINI, Enrico**
**I-41100 Modena (IT)**
• **FAVA, Massimo**
**I-41037 Mirandola (IT)**

(74) Mandataire: **Sutto, Luca et al**
**Gambro Intellectual Property Department,**
**P.O. Box 98**
**41037 Mirandola (MO) (IT)**

(56) Documents cités:
**EP-A- 0 693 297          WO-A-96/08305**
**US-A- 5 098 373          US-A- 5 312 550**
**US-A- 5 510 717**

## Description

La présente invention concerne un système de détermination de la recirculation du sang dans un accès vasculaire.

La présente invention s'applique en particulier à la détermination de la recirculation du sang dans un accès vasculaire lors d'un traitement de dialyse, que l'on prendra comme exemple non limitatif dans la suite de la description.

On sait que le sang se compose d'une partie liquide, le plasma sanguin, et d'une partie corpusculaire formée par des cellules dont les globules rouges qui contiennent l'hémoglobine. En cas d'insuffisance rénale, on trouve, en outre, dans le sang des substances indésirables de bas poids moléculaire qui peuvent être éliminées par un traitement dialytique réalisé au moyen d'un appareil de dialyse.

Les traitements de dialyse présentent une efficacité définie comme le rapport entre le volume de sang purifié au cours de la séance de dialyse et le volume sanguin total du patient.

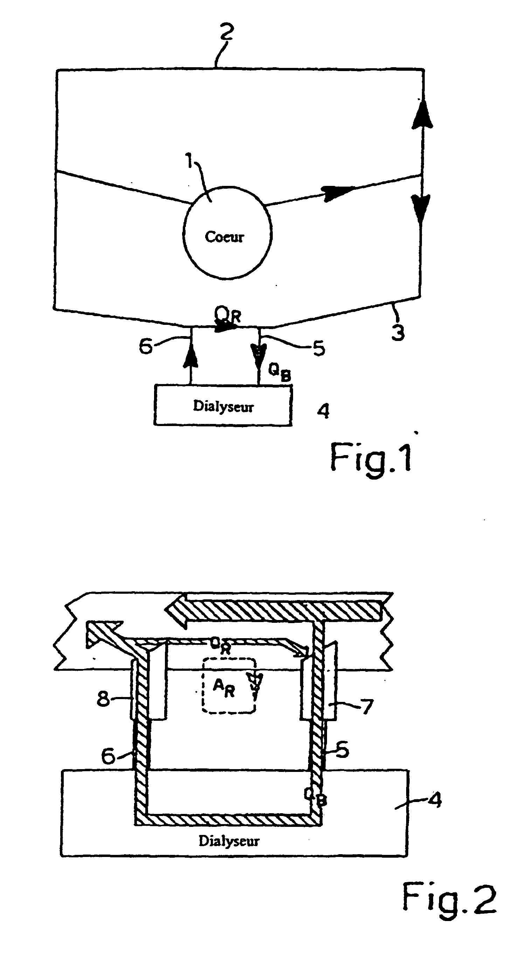

Un modèle simplifié des flux sanguins dans un système patient-circuit extracorporel où la communication entre le patient et le circuit extracorporel se fait au niveau d'un accès vasculaire de type fistule de Cimino-Brescia est illustré sur la figure 1, sur laquelle la référence 1 désigne le coeur, la référence 2 le circuit pulmonaire, la référence 3 le circuit vasculaire ou systémique et la référence 4 un dialyseur relié au circuit systémique 3 par l'intermédiaire d'une ligne de prélèvement de sang 5 (ligne artérielle) et d'une ligne de restitution de sang 6 (ligne veineuse).

Comme le montre la figure 1, le sang traité au cours d'une séance de dialyse provient du circuit systémique 3 dans lequel le sang s'écoule avec un débit limité. De ce fait, les traitements dialytiques actuels présentent une efficacité limitée et il n'existe pas à l'heure actuelle de moyens correcteurs permettant d'en augmenter la valeur.

Par ailleurs, l'efficacité des traitements dialytiques est également réduite par le phénomène connu dans le domaine médical sous le terme de "recirculation dans l'accès vasculaire", dont l'ampleur dépend de multiples facteurs tels que le débit du sang dans le circuit extracorporel, la position des aiguilles et le degré de sténose de la fistule. Il y a recirculation quand une partie du sang traité dans le circuit extracorporel est remise en circulation dans ce circuit par la ligne artérielle 5 aussitôt après avoir été injectée dans l'accès vasculaire 4 par la ligne veineuse 6. Ce phénomène est illustré sur la figure 2 où les références 7 et 8 désignent les aiguilles de prélèvement et de restitution du sang respectivement connectées aux lignes artérielle 5 et veineuse 6.

La valeur AR de la recirculation dans l'accès vasculaire est définie par l'expression suivante:

$$A_R \% = \frac{Q_R}{Q_B} \, 100 \qquad (1)$$

dans laquelle QB est le débit du sang circulant dans le circuit extracorporel et QR est le débit du sang retournant dans le circuit extracorporel par l'intermédiaire de la ligne artérielle 5 immédiatement après le traitement dialytique.

La connaissance de la valeur AR de la recirculation dans l'accès vasculaire présente plusieurs avantages pratiques en ce qui concerne les traitements de dialyse : elle indique que les aiguilles 7, 8 doivent être repositionnées lorsque la valeur AR de la recirculation est trop élevée et elle permet d'agir en vue d'augmenter la précision de la thérapie dialytique, de contrôler à long terme la sténose de la fistule et d'augmenter la durée de vie moyenne de la fistule elle-même.

Pour la détermination de la valeur AR de la recirculation dans l'accès vasculaire, on connaît plusieurs procédés de mesure (voir par exemple EP-A-0 693 297) qui peuvent être regroupés en deux grands groupes, le premier comprenant les procédés de mesure ne faisant pas appel à une sollicitation extérieure et le second faisant appel à une telle sollicitation.

Au premier groupe appartiennent les procédés de mesure qui ne font pas appel à une sollicitation de nature chimique ou physique du sang soumis au traitement de dialyse et qui ne font que quantifier des grandeurs physiologiques au cours de la séance de dialyse.

Appartient par exemple à ce groupe le procédé consistant à mesurer la concentration en urée de trois échantillons de sang prélevés au même moment dans la ligne artérielle, dans la ligne veineuse et dans le circuit vasculaire du patient, et à calculer la valeur AR de la recirculation dans l'accès vasculaire à partir de l'équation suivante (équivalente à l'équation 1) :

$$A_R \% = \frac{C_S - C_A}{C_S - C_V} \, 100 \qquad (2)$$

dans laquelle CS est la valeur de la concentration en urée dans la circulation vasculaire (concentration systémique), CA est la valeur de la concentration en urée dans la ligne artérielle (concentration artérielle) et CV est la valeur de la concentration en urée dans la ligne veineuse (concentration veineuse).

Ce procédé présente l'inconvénient de reposer sur l'hypothèse que, en l'absence de recirculation dans l'accès vasculaire, la valeur de la concentration systémique CS est égale à la valeur de la concentration artérielle CA. Or il a été démontré récemment qu'une telle hypothèse n'est pas valable dans toutes les conditions et qu'elle dépend du point de prélèvement. Il existe donc entre ces valeurs des différences qui compromettent la fiabilité de la mesure, même en l'absence de recirculation dans l'accès vasculaire.

Au second groupe appartiennent les procédés de mesure qui prévoient des sollicitations de nature chimique ou physique du sang soumis à un traitement de dialyse.

Appartient par exemple à ce deuxième groupe le procédé de mesure consistant, comme le précédent, à mesurer la concentration de l'urée dans le sang dans la ligne artérielle, dans la ligne veineuse et dans le circuit vasculaire du patient. Mais, à la différence du procédé mentionné ci-dessus, lors du prélèvement de sang dans la ligne artérielle pour la détermination de la valeur CS de la concentration systémique, le débit QB du sang circulant dans le circuit extracorporel est réglé au minimum afin de limiter au maximum la recirculation dans la fistule et donc de réduire les différences entre les valeurs CS et CA de la concentration systémique et de la concentration artérielle.

Appartient également au deuxième groupe le procédé consistant à imposer au sang du patient un traceur afin d'obtenir une dilution de nature chimique ou physique du sang et à contrôler simultanément, au moyen de capteurs spécifiques, l'évolution du sang dans la ligne artérielle, ou dans la ligne veineuse, ou dans les deux lignes. La comparaison des intégrales des signaux détectés par les capteurs permet de déterminer la valeur AR de la recirculation dans l'accès vasculaire.

En particulier, un premier procédé connu consiste à mesurer la température du sang au moyen de capteurs de température disposés sur la ligne veineuse et sur la ligne artérielle pour surveiller l'allure des températures relatives en réponse à une quantité de chaleur (considérée ici comme un traceur) administrée ou extraite du sang au moyen de l'appareil de dialyse.

Un second procédé de mesure connu fondé sur une dilution du sang est décrit dans le brevet US 5 312 550. Selon ce brevet, il est prévu d'injecter dans la ligne veineuse une substance ayant des propriétés physiques différentes de celles du sang et de détecter la recirculation du sang dans l'accès vasculaire au moyen d'une mesure des propriétés physiques de cette substance en amont du point d'injection de la substance.

Un troisième procédé de mesure connu fondé sur une dilution du sang est décrit dans le brevet US 5 510 717. Selon ce brevet, il est prévu d'injecter un bolus d'une solution hypertonique (traceur) dans la ligne veineuse, et de mesurer la conductivité du sang au moyen de deux capteurs disposés sur la ligne veineuse et sur la ligne artérielle afin de contrôler l'évolution de la conductivité relative en réponse à l'injection du bolus.

Un quatrième procédé de mesure connu fondé sur une dilution du sang consiste à injecter un bolus d'une solution isotonique (traceur) dans la ligne artérielle en amont d'un dispositif de mesure d'absorption optique disposé sur cette ligne. La valeur AR de la recirculation dans l'accès vasculaire est obtenue en comparant la mesure effectuée par le dispositif optique immédiatement après l'injection du bolus avec la mesure qui est effectuée après que le bolus a été partiellement remis en circulation dans le circuit extracorporel par l'accès artériel.

La présente invention a pour but de réaliser un système de détermination de la valeur de la recirculation du sang dans un circuit extracorporel qui soit simple, entièrement automatique et qui permette de réduire les erreurs de mesure et d'utiliser un petit nombre de capteurs, qui font déjà généralement partie d'un appareil de dialyse.

Pour atteindre ce but, on prévoit, conformément à l'invention un système de traitement de sang selon la revendication 1. Les revendications dépendantes 2-16 concernent des modes particuliers de réalisation de l'invention.

D'autres caractéristiques et avantages de l'invention apparaîtront mieux à la lecture de la description qui va suivre. On se reportera aux dessins annexés, sur lesquels:

- la figure 1 représente un modèle simplifié des flux sanguins qui ont leur origine dans l'organisme d'un patient relié à un circuit pour circulation extracorporelle de sang;
- la figure 2 est un schéma simplifié d'un accès vasculaire et des flux sanguins qui y circulent;
- la figure 3 est un schéma simplifié d'un système de dialyse;
- la figure 4 est un organigramme des opérations effectuées par le système de la figure 3 pour mettre en oeuvre le procédé selon l'invention; et
- les figures 5, 6, 7 sont des diagrammes représentant l'évolution par rapport au temps de grandeurs mesurées ou contrôlées durant la mise en oeuvre du procédé selon l'invention.

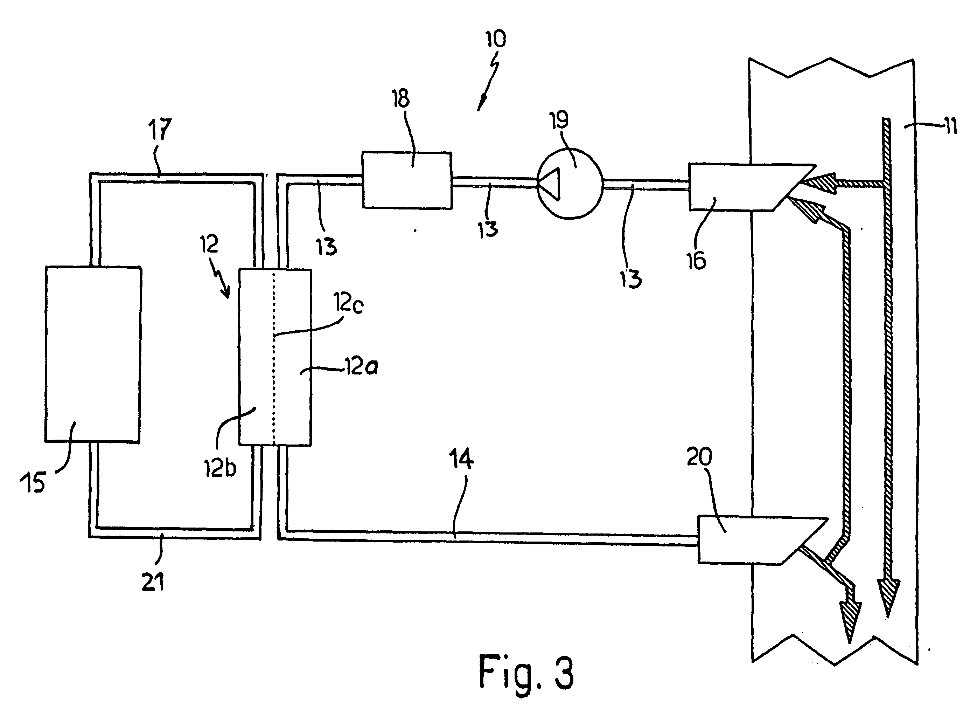

Sur la figure 3, la référence 10 désigne un circuit extracorporel pour le traitement du sang relié à une fistule 11 (accès vasculaire) d'un patient soumis à un traitement de dialyse. La figure 3 ne représente que les éléments d'un système de dialyse utiles à la compréhension du procédé selon l'invention.

Le circuit extracorporel 10 comprend un dialyseur 12 ayant un compartiment sang 12a et un compartiment pour liquide de dialyse 12b séparés par une membrane 12c semi-perméable. Une ligne artérielle 13 relie la fistule 11 du patient à une entrée du compartiment sang 12a et une ligne veineuse 14 relie une sortie du compartiment sang 12a à la fistule 11. La ligne artérielle 13 et la ligne veineuse 14 sont connectées à la fistule 11 par l'intermédiaire de canules 16, 20, la canule artérielle 16 étant implantée en amont de la canule veineuse 20 par rapport au sens de circulation du sang dans la fistule 11. Sur la ligne artérielle 13 sont disposées, dans le sens de circulation du sang, une pompe à sang 19 et un dispositif de mesure de l'hémoglobine 18.

Le compartiment pour liquide de dialyse 12b du dialyseur 12 a une entrée et une sortie reliées à une machine de dialyse 15, respectivement par l'intermédiaire d'une canalisation d'alimentation en liquide de dialyse frais 17 et une canalisation d'évacuation en liquide usé 21. La machine de dialyse 15 comprend notamment une pompe d'ultrafiltration (non représentée) permettant de provoquer le passage d'eau plasmatique au travers de la membrane 12c du dialyseur 12.

Le système de dialyse représenté sur la figure 3 comprend également une unité de calcul et de commande. Cette unité est reliée à une interface utilisateur par laquelle elle reçoit des instructions, telles que diverses valeurs de consigne. Par ailleurs, elle reçoit des informations émises par les organes de mesure du système, tel que le dispositif de mesure d'hémoglobine 18. Elle pilote, en fonction des instructions reçues et de modes d'opération et d'algorithmes programmés les organes moteurs du système, tels que la pompe à sang 19 et la pompe d'ultrafiltration.

La figure 3 met par ailleurs en évidence les flux de sang dans l'accès vasculaire.

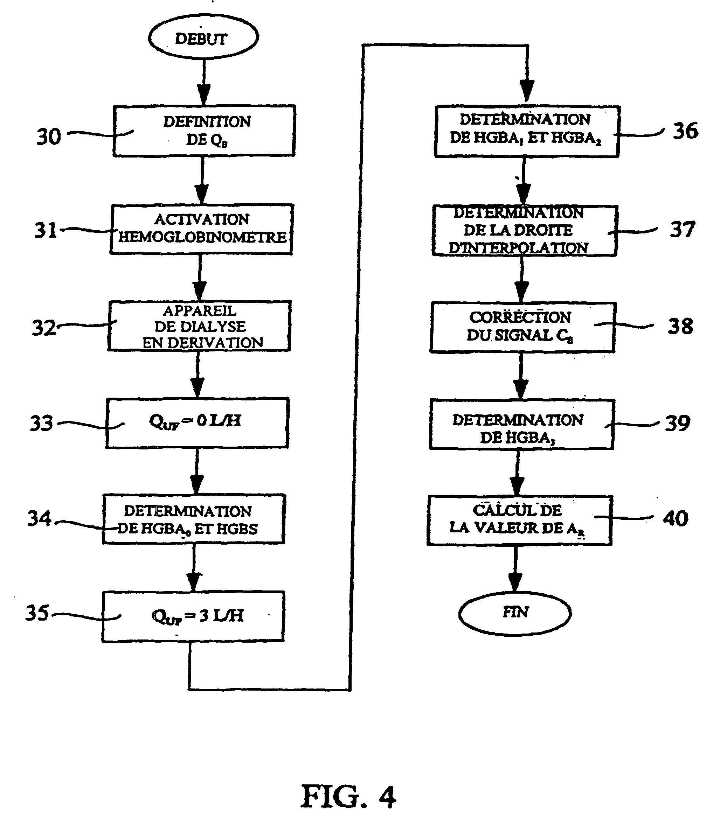

Lorsque le système de dialyse est en fonctionnement, les opérations décrites ci-dessous en référence à la figure 4 sont exécutées.

Le procédé de détermination de la recirculation du sang selon l'invention est fondé sur une variation d'une caractéristique du sang provoquée par la pompe d'ultrafiltration, à savoir la concentration du sang.

Au début de la séance de dialyse, comme le montre la figure 4, on impose une valeur QB au débit de la pompe à sang 19 (bloc 30).

L'hémoglobinomètre 18 est ensuite activé pour l'acquisition et la mémorisation du signal de concentration CE durant la totalité de la mesure nécessaire à la détermination de la recirculation (bloc 31).

Ensuite, la circulation du liquide de dialyse dans le dialyseur 12 est interrompue (bloc 32) de sorte qu'après une période transitoire initiale le liquide de dialyse dans le dialyseur a la même concentration électrolytique que le sang. La circulation du liquide de dialyse dans le dialyseur 12 n'est rétablie que lorsque les mesures nécessaire à la détermination de la recirculation ont été effectuées.

Cette opération permet de limiter l'influence indésirable des éventuels déséquilibres osmotiques sur la concentration en hémoglobine en raison desquels, même en l'absence d'ultrafiltration, il se produit en général une variation de la valeur de concentration en hémoglobine du sang due aux déplacements de l'eau contenue dans les cellules sanguines et dans les espaces interstitiels.

La pompe d'ultrafiltration est ensuite commandée pour que la valeur QUF du débit d'ultrafiltration dans le dialyseur 12 soit sensiblement nulle (bloc 33); il en résulte que les valeurs HGBA et HGBV des concentrations en hémoglobine dans la ligne artérielle 13 et dans la ligne veineuse 14 sont égales.

On détermine ensuite, en fonction du signal de concentration CE, la valeur instantanée HGBA0 de la concentration en hémoglobine dans la ligne artérielle 13, qui est égale à la valeur HGBS de la concentration systémique en hémoglobine lorsque le débit d'ultrafiltration est nul (bloc 34). On impose ainsi l'égalité suivante HGBS = HGBA0.

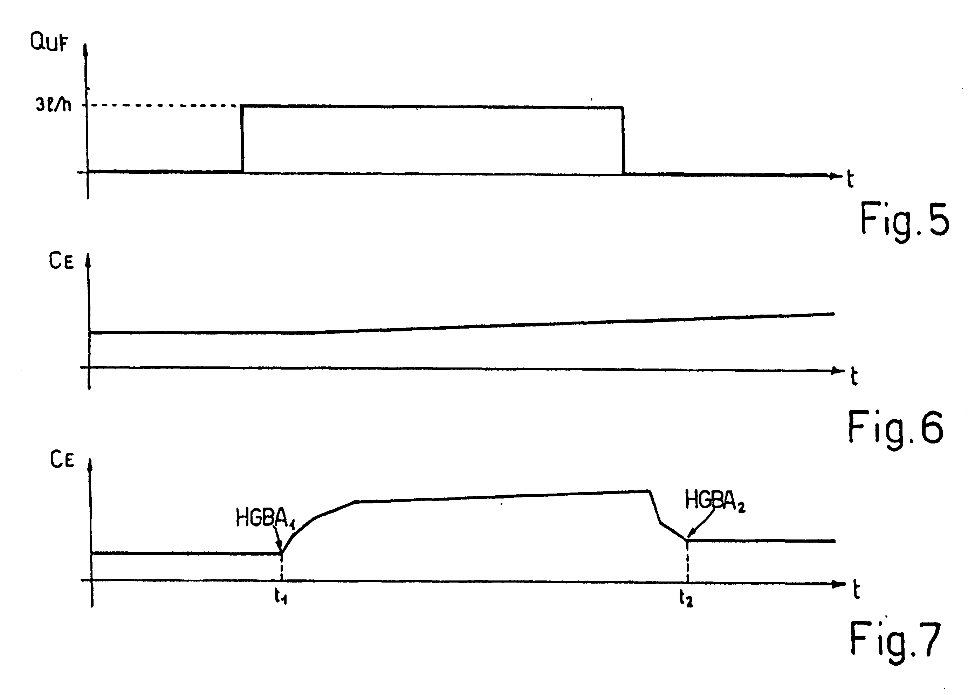

La pompe d'ultrafiltration est ensuite commandée pour que le débit d'ultrafiltration QUF soit égal à une valeur déterminée pendant un intervalle de temps déterminé. Par exemple, on impose un débit d'ultrafiltration QUF égal à 3 l/h pendant 5 minutes (bloc 35); pour cet exemple, l'évolution du débit d'ultrafiltration QUF par rapport au temps est représentée sur le diagramme de la figure 5.

La première variation du débit d'ultrafiltration QUF, de zéro à la valeur déterminée, a pour conséquence une augmentation de la valeur de la concentration en hémoglobine du sang réintroduit dans la fistule 11 du patient par l'intermédiaire de la ligne veineuse 14, tandis que la seconde variation du débit d'ultrafiltration QUF, de la valeur déterminée à zéro, provoque une décroissance de la concentration en hémoglobine jusqu'à un niveau proche de la valeur initiale de l'hémoglobine.

A partir du signal de concentration CE fournit par l'hémoglobinomètre 18, il est possible de déduire la valeur de recirculation dans la fistule 11.

S'il n'y a pas de recirculation dans l'accès vasculaire, le sang concentré dans le dialyseur 12 lors de l'épisode d'ultrafiltration et injecté dans l'organisme du patient par l'intermédiaire de la ligne veineuse 14 parcourt un long trajet à l'intérieur du corps du patient avant de circuler à nouveau dans le dialyseur 12.

Le sang concentré est soumis durant ce trajet à une telle dilution que le signal de concentration CE produit par l'hémoglobinomètre 18 ne révèle initialement aucune augmentation de la concentration en hémoglobine dans la ligne artérielle 13 (comme le montre le diagramme de la figure 6), et il n'augmentera légèrement que quelques minutes après le début du palier de débit d'ultrafiltration (non illustré sur la figure 6 car extérieur à l'intervalle de temps représenté).

Si au contraire il y a recirculation dans l'accès vasculaire 11, une partie du sang concentré injecté dans le corps du patient est immédiatement réintroduit dans la ligne artérielle 13 et le signal de concentration CE fournit par l'hémoglobinomètre 18 augmente rapidement, comme le montre le diagramme de la figure 7. Dans l'exemple considéré, cette augmentation peut être observée environ une minute après la création d'un régime d'ultrafiltration et elle atteint une valeur sensiblement constante après environ trois minutes.

On calcule la valeur AR de la recirculation dans la fistule 11 à partir de la valeur du signal de concentration CE prise en régime stabilisé (blocs 36 à 40).

Le calcul de la valeur AR de la recirculation dans la fistule 11 est effectué en tenant compte du phénomène de "remplissage plasmatique" selon lequel, lorsque de l'eau plasmatique est soustraite du sang par ultrafiltration, l'organisme du patient soumis à ce traitement réagit et alimente le sang avec de l'eau prélevée dans les cellules du sang lui-même et dans les espaces interstitiels.

En effet, le signal de concentration CE produit par l'hémoglobinomètre 18 à la suite de la première variation du débit d'ultrafiltration comprend une composante principale correspondant à .l'amplitude de l'hémofiltration imposée et une composante, croissant en général de manière linéaire dans le temps, correspondant à la différence entre le débit d'ultrafiltration et au débit du remplissage plasmatique.

Pour éliminer cette composante, en fonction de la valeur du signal de concentration CE mémorisé, on détermine les valeurs HGBA1 et HGBA2 prises par la concentration en hémoglobine dans la ligne artérielle 13 aux temps, notés t1 et t2 sur la figure 7, auxquels elle commence à augmenter au début de l'épisode d'ultrafiltration et finit de décroître à la fin de l'épisode d'ultrafiltration, respectivement (bloc 36).

Lorsque le débit de remplissage plasmatique est égal au débit d'ultrafiltration, les valeurs HGBA1 et HGBA2 sont égales. En revanche, les valeurs HGBA1 et HGBA2 sont différentes lorsque le débit de remplissage est nul ou est inférieur au débit d'ultrafiltration, HGBA2 étant supérieur à HGBA1.

On détermine alors l'équation de la droite d'interpolation passant par les points HGBA1 et HGBA2 de la figure 7, qui représente l'évolution par rapport au temps de la composante indésirable du signal de concentration CE, cette composante croissant généralement de façon linéaire en fonction du temps (bloc 37).

Ensuite, le signal de concentration CE est corrigé de l'effet du phénomène de remplissage plasmatique par soustraction de la droite d'interpolation au signal de concentration CE produit par l'hémoglobinomètre 18 pendant l'épisode d'hémofiltration et est mis en mémoire dans l'unité de commande et de calcul (bloc 38).

On détermine alors la valeur HGBA3 de la concentration en hémoglobine dans la ligne artérielle 13 en fonction de la valeur prise en régime établi par la concentration CE corrigée. Par exemple, cette valeur peut être la valeur mesurée à un instant déterminé (par exemple 3 minutes) suivant le début de l'épisode d'ultrafiltration (bloc 39).

La valeur HGBA3 de la concentration en hémoglobine dans la ligne artérielle 13 peut être aussi calculée de la façon suivante: à partir d'une série de valeurs de la concentration du sang échantillonnées à partir de t1, l'unité de calcul détermine l'équation de la courbe représentant l'évolution de la concentration du sang, puis en calcule l'asymptote. La concentration en hémoglobine HGBA3 est choisie égale à cette asymptote. L'intérêt de ce mode de détermination de HGBA3 est qu'il permet de réduire sensiblement la durée de l'épisode d'hémofiltration.

Enfin on calcule la valeur AR de la recirculation dans la fistule 11 à l'aide de l'équation suivante (bloc 40) :

$$A_R \% = \frac{Q_B - Q_{UF}}{Q_B + Q_{UF} \left( \dfrac{HGBS}{HGBA_3 - HGBS} \right)} \cdot 100$$

dans laquelle QB est le débit de la pompe à sang 19 (imposé au bloc 30), QUE est le débit d'ultrafiltration (imposé au bloc 35), HGBS est la concentration systémique (calculée au bloc 34) et HGBA3 est la concentration artérielle (calculée au bloc 39).

Les avantages du présent procédé de mesure sont les suivants.

Avant tout, le présent procédé est simple à mettre en oeuvre, contrairement aux procédés qui ne reposent pas sur une sollicitation extérieure, qui nécessitent des prélèvements sanguins et des examens de laboratoire, et aussi contrairement aux procédés qui reposent sur une sollicitation extérieure, qui nécessitent des interventions manuelles (injection de solution saline).

En outre, le présent procédé peut être mis en oeuvre de manière entièrement automatique.

En outre, le procédé utilise un seul capteur, l'hémoglobinomètre 18, au lieu de deux comme bon nombre des procédés décrits ci-dessus, et il permet donc de réduire les erreurs de mesure.

Enfin, le procédé ne nécessite aucune modification de l'appareil de dialyse 10 dans la mesure où les systèmes de dialyse courant comportent généralement un hémoglobinomètre 18 et une pompe d'ultrafiltration 15.

Finalement, il apparaît clairement qu'il est possible d'apporter au procédé décrit et illustré ici des modifications et des variantes sans pour autant sortir du cadre de la présente invention.

Par exemple, dans une variante du procédé, le débit d'ultrafiltration initial a une valeur déterminée différente de zéro et la variation du débit d'ultrafiltration provoquée pour mesurer la recirculation du sang dans l'accès vasculaire peut être positive ou négative (augmentation ou diminution du débit d'ultrafiltration d'une quantité déterminée).

Dans ce cas, le procédé prévoit:

- de mesurer la valeur instantanée HGBA0 de la concentration en hémoglobine dans la ligne artérielle 13 lorsque le débit d'ultrafiltration est égal à sa valeur déterminée initiale,
- de commander un palier négatif du débit d'ultrafiltration,
- de déterminer la valeur HGBA3 de la concentration en hémoglobine dans la ligne artérielle 13,
- d'égaler la valeur HGBS de la concentration systémique en hémoglobine à la valeur HGBA3 lorsque le débit d'ultrafiltration prend une valeur nulle au cours de cette étape (HGBS = HGBA3),

- de corriger cette valeur en fonction de la droite d'interpolation de la manière décrite ci-dessus, et

- de calculer la valeur AR de la recirculation dans l'accès vasculaire à l'aide de l'équation suivante:

$$A_R \% = \frac{Q_B - Q_{UF}}{Q_B + Q_{UF} \left(\frac{HGBS}{HGBA_0 - HGBS}\right)} \ 100$$

En outre, le présent procédé peut être aussi mis en oeuvre, avec une précision comparable, lorsque le phénomène de remplissage plasmatique donne lieu, du fait de divers facteurs tels que les mouvements et les déséquilibres hydriques et osmotiques, à un apport d'eau dans le sang supérieur à l'eau plasmatique ultrafiltrée, c'est-à-dire à une diminution de la concentration en hémoglobine dans le sang. Dans ce cas, la composante indésirable du signal de concentration produit par l'hémoglobinomètre 18 présente une allure décroissant de façon linéaire dans le temps.

## Revendications

1. Système de traitement du sang comprenant:

   - un appareil (12) à membrane semi-perméable pour le traitement du sang;
   - une ligne artérielle destinée à interconnecter un accès vasculaire (11) d'un patient à une entrée de l'appareil de traitement (12),
   - une ligne veineuse (14) destinée à interconnecter une sortie de l'appareil de traitement (12) à l'accès vasculaire (11),
   - des moyens pour provoquer une filtration d'eau plasmatique au travers de la membrane de l'appareil de traitement (12);
   - des moyens pour déterminer un paramètre significatif de la concentration du sang disposés sur la ligne artérielle (13);

   **caractérisé en ce qu'**il comporte des moyens de calcul et de commande pour:

   • piloter les moyens pour provoquer la filtration d'eau plasmatique de façon à engendrer une variation momentanée de la concentration du sang dans l'appareil de traitement (12) en réduisant ou augmentant momentanément la proportion d'eau plasmatique du sang mis en circulation dans l'appareil à membrane,
   • mettre en mémoire des valeurs du paramètre significatif de la concentration du sang, et
   • calculer la recirculation du sang à partir des valeurs prises par le paramètre en réponse à un pilotage déterminé des moyens pour provoquer une concentration du sang.

2. Système selon la revendication 1, **caractérisé en ce qu'**il comprend :

   - des moyens (19) pour régler le débit du sang dans le circuit extracorporel à une valeur déterminée ($Q_B$);
   - des moyens (18) pour déterminer une première valeur (HGBA0) du paramètre significatif de la concentration du sang dans le circuit extracorporel;
   - des moyens pour provoquer une première variation d'amplitude déterminée ($Q_{UF}$) du débit d'ultrafiltration dans l'appareil de traitement (12);
   - des moyens (18) pour déterminer une deuxième valeur (HGBA3) du paramètre significatif de la concentration du sang dans la ligne artérielle (13);
   - des moyens pour calculer la recirculation (AR) du sang dans le circuit extracorporel en fonction du débit du sang ($Q_B$), de l'amplitude déterminée ($Q_{UF}$) de la première variation du débit d'ultrafiltration et des première et deuxième valeurs (HGBA0, HGBA3) du paramètre significatif de la concentration du sang dans la ligne artérielle (13).

3. Système selon la revendication 2, **caractérisé en ce que** les moyens pour déterminer une deuxième valeur (HGBA3) du paramètre significatif de la concentration du sang dans la ligne artérielle comprennent:

   - des moyens pour provoquer une deuxième variation du débit d'ultrafiltration dans l'appareil de traitement d'amplitude opposée à l'amplitude de la première variation;
   - des moyens (18) pour déterminer une troisième et une quatrième valeurs (HGBA1, HGBA2) du paramètre significatif de la concentration du sang dans la ligne artérielle (13) avant la première variation du débit d'ultrafiltration et après la deuxième variation du débit d'ultrafiltration, respectivement;
   - des moyens pour déterminer une droite d'interpolation entre la troisième et la quatrième valeurs (HGBA1, HGBA2) du paramètre significatif de la concentration du sang;
   - des moyens pour déterminer une valeur en régime établi du paramètre significatif de la concentration du sang dans la ligne artérielle après la première variation du débit d'ultrafiltration;
   - des moyens pour corriger la valeur de régime établi du paramètre significatif de la concentration du sang dans la ligne artérielle (13) en fonction de la droite d'interpolation pour obtenir la deuxième valeur (HGBA3) du paramètre significatif de la concentration du sang dans la ligne artérielle.

4. Système selon la revendication 3, **caractérisé en ce que** les moyens (18) pour déterminer un paramètre significatif de la concentration du sang sont prévus pour déterminer:

   - la troisième valeur (HGBA1) du paramètre significatif de la concentration du sang dans la ligne artérielle (13) au moment où la concentration du sang commence à varier en réponse à la première variation du débit d'ultrafiltration et
   - la quatrième valeur (HGBA2) du paramètre significatif de la concentration du sang dans la ligne artérielle (13) au moment où la concentration du sang finit de varier en réponse à la deuxième variation du débit d'ultrafiltration.

5. Système selon une des revendications 2 à 4, **caractérisé en ce que** les moyens (18) pour déterminer un paramètre significatif de la concentration du sang sont prévus pour déterminer la deuxième valeur (HGBA3) du paramètre significatif de la concentration du sang dans la ligne artérielle (13) après un temps déterminé après le début de la première variation du débit d'ultrafiltration.

6. Système selon la revendications 5, **caractérisé en ce que** les moyens (18) pour déterminer un paramètre significatif de la concentration du sang sont prévus pour déterminer la deuxième valeur (HGBA3) du paramètre significatif de la concentration du sang dans la ligne artérielle (13) en régime stabilisé après le début de la première variation du débit d'ultrafiltration.

7. Système selon une des revendications 2 à 6, **caractérisé en ce que** les moyens pour provoquer une première variation du débit d'ultrafiltration, préalablement à l'étape de provoquer une première variation du débit d'ultrafiltration, règlent le débit d'ultrafiltration dans l'appareil de traitement sensiblement égal à zéro.

8. Système selon la revendication 7, **caractérisé en ce que** les moyens pour provoquer une première variation d'amplitude prédéterminée ($Q_{UF}$) du débit d'ultrafiltration dans l'appareil de traitement sont prévus pour augmenter le débit d'ultrafiltration.

9. Système selon la revendication 8, **caractérisé en ce que** les moyens pour calculer la recirculation (AR) du sang dans le circuit extracorporel sont prévus pour résoudre l'équation suivante:

$$A_R \% = \frac{Q_B - Q_{UF}}{Q_B + Q_{UF}\left(\dfrac{HGBS}{HGBA_3 - HGBS}\right)} \, 100$$

où:

   - $Q_B$ est le débit du sang dans le circuit extracorporel;
   - $Q_{UF}$ est l'amplitude prédéterminée de la première variation du débit d'ultrafiltration ;
   - HGBS est la valeur de la concentration systémique du sang, égale à la première valeur (HGBA0) du paramètre significatif de la concentration du sang dans la ligne artérielle (13); et
   - HGBA3 est la deuxième valeur du paramètre significatif de la concentration du sang dans la ligne artérielle (13).

10. Système selon une des revendications 2 à 6, **caractérisé en ce que** les moyens pour provoquer une première variation d'amplitude prédéterminée ($Q_{UF}$) du débit d'ultrafiltration dans l'appareil de traitement sont prévus pour réduire un débit d'ultrafiltration existant jusqu'à ce qu'il devienne sensiblement égal à zéro.

11. Système selon la revendication 10, **caractérisé en ce que** les moyens pour calculer la recirculation (AR) du sang dans le circuit extracorporel sont prévus pour appliquer la formule suivante:

$$A_R \% = \frac{Q_B - Q_{UF}}{Q_B + Q_{UF}\left(\dfrac{HGBS}{HGBA_0 - HGBS}\right)} \, 100$$

où:

   - $Q_B$ est le débit du sang dans le circuit extracorporel;
   - $Q_{UF}$ est l'amplitude prédéterminée de la première variation du débit d'ultrafiltration;
   - HGBA0 est la première valeur du paramètre significatif de la concentration du sang dans la ligne artérielle (13) ;
   - HGBS est la valeur de la concentration systémique du sang, égale à la deuxième valeur (HGBA3) du paramètre significatif de la concentration du sang dans la ligne artérielle (13).

12. Système selon une des revendications 1 à 11, **caractérisé en ce que** l'appareil de traitement est un dialyseur (12) dans lequel un liquide de dialyse peut être mis en circulation, et **en ce que** le système comprend des moyens (15) pour interrompre la circulation du liquide de dialyse préalablement à l'étape de déterminer une première valeur du paramètre significatif de la concentration (HGBA0) du sang dans le circuit extracorporel.

13. Système selon une des revendications 1 à 12, **caractérisé en ce que** le système comporte des moyens (15) pour rétablir la circulation du liquide de dialyse lorsque les mesures nécessaires à la détermination de la recirculation ont été effectuées.

14. Système selon une des revendications 1 à 13, **caractérisé en ce que** les moyens (18) pour déterminer un paramètre significatif de la concentration du sang sur la ligné artérielle sont prévus pour acquérir et pour mémoriser le signal de concentration durant la totalité de la mesure nécessaire à la détermination de la recirculation.

15. Système selon une des revendications 1 à 9, **caractérisé en ce que** les moyens pour provoquer une première variation du débit d'ultrafiltration sont prévus pour imposer un débit d'ultrafiltration égal à trois litres par heure pendant cinq minutes.

16. Système selon une des revendications 1 à 15, **caractérisé en ce que** le paramètre significatif de la concentration du sang est la concentration du sang en hémoglobine.


**Claims**

1. Blood treatment system comprising:

   - a blood treatment apparatus (12) with semipermeable membrane;
   - an arterial line, intended to interconnect a vascular access (11) of a patient with an inlet of the treatment apparatus (12);
   - a venous line (14) intended to interconnect an outlet of the treatment apparatus (12) with the vascular access (11);
   - means for inducing plasma fluid filtration through the membrane of the treatment apparatus (12);
   - means for determining a parameter signifying the blood concentration and are arranged on the arterial line (13);

   **characterized in that** it includes computation and control means for:

   • driving the means for inducing plasma fluid filtration in such a way as to generate a momentary variation of the blood concentration in the treatment apparatus (12) by momentarily reducing or increasing the proportion of blood plasma fluid circulating in the membrane apparatus,
   • storing values of the parameter signifying the blood concentration, and
   • calculating the blood recirculation on the basis of the values which the parameter takes in response to a determined drive programme of the means for inducing a blood concentration.

2. System according to Claim 1, **characterized in that** it comprises:

   - means (19) for setting the blood flow rate in the extracorporeal circuit to a determined value $(Q_B)$;
   - means (18) for determining a first value (HGBA0) of the parameter signifying the blood concentration in the extracorporeal circuit;
   - means for inducing a first variation, of determined amplitude $(Q_{UF})$, of the ultrafiltration rate in the treatment apparatus (12);
   - means (18) for determining a second value (HGBA3) of the parameter signifying the blood concentration in the arterial line (13);
   - means for calculating the blood recirculation (AR) in the extracorporeal circuit as a function of the blood flow rate $(Q_B)$, of the determined amplitude $(Q_{UF})$ of the first variation of the ultrafiltration rate, and of the first and second values (HGBA0, HGBA3) of the parameter signifying the blood concentration in the arterial line (13).

3. System according to Claim 2, **characterized in that** the means for determining a second value (HGBA3) of the parameter signifying the blood concentration in the arterial line comprise:

   - means for inducing a second variation of the ultrafiltration rate in the treatment apparatus, with the opposite amplitude to the amplitude of the first variation;
   - means (18) for determining a third and a fourth value (HGBA1, HGBA2) of the parameter signifying the blood concentration in the arterial line (13), before the first variation of the ultrafiltration rate and after the second variation of the ultrafiltration rate, respectively;
   - means for determining an interpolation line between the third and fourth values (HGBA1, HGBA2) of the parameter signifying the blood concentration;
   - means for determining a steady-state value of the parameter signifying the blood concentration in the arterial line after the first variation of the ultrafiltration rate;
   - means for correcting the steady-state value of the parameter signifying the blood concentration in the arterial line (13), on the basis of the interpolation line, in order to obtain the second value (HGBA3) of the parameter signifying the blood concentration in the arterial line.

4. System according to Claim 3, **characterized in that** the means (18) for determining a parameter signifying the blood concentration are designed to determine:

- the third value (HGBA1) of the parameter signifying the blood concentration in the arterial line (13) at the time when the blood concentration begins to vary in response to the first variation of the ultrafiltration rate, and
- the fourth value (HGBA2) of the parameter signifying the blood concentration in the arterial line (13) at the time when the blood concentration finishes varying in response to the second variation of the ultrafiltration rate.

5. System according to one of Claims 2 to 4, **characterized in that** the means (18) for determining a parameter signifying the blood concentration are designed to determine the second value (HGBA3) of the parameter signifying the blood concentration in the arterial line (13) following a determined delay after the start of the first variation of the ultrafiltration rate.

6. System according to Claim 5, **characterized in that** the means (18) for determining a parameter signifying the blood concentration are designed to determine the second value (HGBA3) of the parameter signifying the blood concentration in the arterial line (13) in steady state after the start of the first variation of the ultrafiltration rate.

7. System according to one of Claims 2 to 6, **characterized in that** the means for inducing a first variation of the ultrafiltration rate, prior to the step of inducing a first variation of the ultrafiltration rate, set the ultrafiltration rate in the treatment apparatus substantially equal to zero.

8. System according to Claim 7, **characterized in that** the means for inducing a first variation, of predetermined amplitude ($Q_{UF}$), of the ultrafiltration rate in the treatment apparatus are designed to increase the ultrafiltration rate.

9. System according to Claim 8, **characterized in that** the means for calculating the blood recirculation (AR) in the extracorporeal circuit are designed to solve the following equation:

$$A_R \% = \frac{Q_B - Q_{UF}}{Q_B + Q_{UF}(\frac{HGBS}{HGBA_3 - HGBS})} \; 100$$

where:

- $Q_B$ is the blood flow rate in the extracorporeal circuit;
- $Q_{UF}$ is the predetermined amplitude of the first variation of the ultrafiltration rate;
- HGBS is the value of the systemic blood concentration, equal to the first value (HGBA0) of the parameter signifying the blood concentration in the arterial line (13); and
- HGBA3 is the second value of the parameter signifying the blood concentration in the arterial line (13).

10. System according to one of Claims 2 to 6, **characterized in that** the means for inducing a first variation, of predetermined amplitude ($Q_{UF}$), of the ultrafiltration rate in the treatment apparatus are designed to reduce an existing ultrafiltration rate until it becomes substantially equal to zero.

11. System according to Claim 10, **characterized in that** the means for calculating the blood recirculation (AR) in the extracorporeal circuit are designed to apply the following formula:

$$A_R \% = \frac{Q_B - Q_{UF}}{Q_B + Q_{UF}(\frac{HGBS}{HGBA_0 - HGBS})} \; 100$$

where:

- $Q_B$ is the blood flow rate in the extracorporeal circuit;
- $Q_{UF}$ is the predetermined amplitude of the first variation of the ultrafiltration rate;
- HGBA0 is the first value of the parameter signifying the blood concentration in the arterial line (13);
- HGBS is the value of the systemic blood concentration, equal to the second value (HGBA3) of the parameter signifying the blood concentration in the arterial line (13).

12. System according to one of Claims 1 to 11, **characterized in that** the treatment apparatus is a dialyser (12) in which a dialysis liquid can be circulated, and **in that** the system comprises means (15) for interrupting the circulation of the dialysis liquid prior to the step of determining a first value (HGBA0) of the parameter signifying the blood concentration in the extracorporeal circuit.

**13.** System according to one of Claims 1 to 12, **characterized in that** the system includes means (15) for reestablishing circulation of the dialysis liquid when the measurements needed for determining the recirculation have been made.

**14.** System according to one of Claims 1 to 13, **characterized in that** the means (18) for determining a parameter signifying the blood concentration in the arterial line are designed to acquire and store in memory the concentration signal throughout the measurement needed for determining the recirculation.

**15.** System according to one of Claims 1 to 9, **characterized in that** the means for inducing a first variation of the ultrafiltration rate are designed to impose an ultrafiltration rate of three litres per hour for five minutes.

**16.** System according to one of Claims 1 to 15, **characterized in that** the parameter signifying the blood concentration is the blood haemoglobin concentration.

**Patentansprüche**

**1.** System zur Behandlung von Blut, das Folgendes umfasst:

- eine Apparatur (12) mit semipermeabler Membran zur Behandlung von Blut,
- einen arteriellen Schlauch, der zur Verbindung eines Gefäßzugangs (11) eines Patienten mit einem Eingang der Behandlungsapparatur (12) bestimmt ist,
- einen venösen Schlauch (14), der zur Verbindung eines Ausgangs der Behandlungsapparatur (12) mit dem Gefäßzugang (11) bestimmt ist,
- Vorrichtungen zum Bewirken einer Filtration von Plasmawasser durch die Membran der Behandlungsapparatur (12) hindurch,
- Vorrichtungen zur Bestimmung eines signifikanten Parameters der Blutkonzentration, die in den arteriellen Schlauch (13) eingebracht sind,

  **dadurch gekennzeichnet, dass** es Berechnungs- und Steuerungseinheiten umfasst, um:

- die Vorrichtungen zum Bewirken einer Filtration von Plasmawasser so zu steuern, dass eine momentane Veränderung der Blutkonzentration in der Behandlungsapparatur (12) durch momentanes Verringern oder Erhöhen des Anteils an Blutplasmawasser, der in der Membranapparatur in Zirkulation gebracht wird, bewirkt wird,

- Werte des signifikanten Parameters der Blutkonzentration zu speichern und
- die Rezirkulation von Blut ausgehend von den Werten zu berechnen, die durch den Parameter in Abhängigkeit von einer bestimmten Steuerung der Vorrichtungen zum Bewirken einer Blutkonzentration erhalten werden.

**2.** System nach Anspruch 1, **dadurch gekennzeichnet, dass** es Folgendes umfasst:

- Vorrichtungen (19) zum Steuern des Blutdurchsatzes im extrakorporalen Kreislauf auf einen bestimmten Wert ($Q_B$),
- Vorrichtungen (18) zur Bestimmung eines ersten Werts (HGBA0) des signifikanten Parameters der Blutkonzentration im extrakorporalen Kreislauf,
- Vorrichtungen zum Bewirken einer ersten Veränderung der bestimmten Amplitude ($Q_{UF}$) des Ultrafiltrationsdurchsatzes in der Behandlungsapparatur (12),
- Vorrichtungen (18) zur Bestimmung eines zweiten Wertes (HGBA3) des signifikanten Parameters der Blutkonzentration im arteriellen Schlauch (13),
- Vorrichtungen zur Berechnung der Rezirkulation (AR) von Blut im extrakorporalen Kreislauf in Abhängigkeit vom Blutdurchsatz ($Q_B$), der bestimmten Amplitude ($Q_{UF}$) der ersten Veränderung des Ultrafiltrationsdurchsatzes und von dem ersten und zweiten Wert (HGBA0, HGBA3) des signifikanten Parameters der Blutkonzentration im arteriellen Schlauch (13).

**3.** System nach Anspruch 2, **dadurch gekennzeichnet, dass** die Vorrichtungen zur Bestimmung eines zweiten Wertes (HGBA3) des signifikanten Parameters der Blutkonzentration im arteriellen Schlauch Folgendes umfassen:

- Vorrichtungen zum Bewirken einer zweiten Veränderung des Ultrafiltrationsdurchsatzes in der Behandlungsapparatur mit einer zur Amplitude der ersten Veränderung entgegengesetzten Amplitude,
- Vorrichtungen (18) zur Bestimmung eines dritten und eines vierten Wertes (HGBA1, HGBA2) des signifikanten Parameters der Blutkonzentration im arteriellen Schlauch (13) vor der ersten Veränderung des Ultrafiltrationsdurchsatzes bzw. nach der zweiten Veränderung des Ultrafiltrationsdurchsatzes,
- Vorrichtungen zur Bestimmung einer Interpolationsgeraden zwischen dem dritten und vierten Wert (HGBA1, HGBA2) des signifikanten Parameters der Blutkonzentration,

- Vorrichtungen zur Bestimmung eines Wertes im bestehenden Zustand des signifikanten Parameters der Blutkonzentration im arteriellen Schlauch nach der ersten Veränderung des Ultrafiltrationsdurchsatzes,
- Vorrichtungen zum Korrigieren des Wertes des bestehenden Zustands des signifikanten Parameters der Blutkonzentration im arteriellen Schlauch (13) in Abhängigkeit von der Interpolationsgeraden, um den zweiten Wert (HGBA3) des signifikanten Parameters der Blutkonzentration im arteriellen Schlauch zu erhalten.

4. System nach Anspruch 3, **dadurch gekennzeichnet, dass** die Vorrichtungen (18) zur Bestimmung eines signifikanten Parameters der Blutkonzentration bereitgestellt werden, um Folgendes zu bestimmen:

- den dritten Wert (HGBA1) des signifikanten Parameters der Blutkonzentration im arteriellen Schlauch (13) in dem Moment, in dem die Blutkonzentration in Reaktion auf die erste Veränderung des Ultrafiltrationsdurchsatzes sich zu verändern beginnt und
- den vierten Wert (HGBA2) des signifikanten Parameters der Blutkonzentration im arteriellen Schlauch (13) in dem Moment, in dem die Blutkonzentration in Reaktion auf die zweite Veränderung des Ultrafiltrationsdurchsatzes sich zu verändern aufhört.

5. System nach einem der Ansprüche 2 bis 4, **dadurch gekennzeichnet, dass** die Vorrichtungen (18) zur Bestimmung eines signifikanten Parameters der Blutkonzentration bereitgestellt werden, um den zweiten Wert (HGBA3) des signifikanten Parameters der Blutkonzentration im arteriellen Schlauch (13) nach einer bestimmten Zeit nach Beginn der ersten Veränderung des Ultrafiltrationsdurchsatzes zu bestimmen.

6. System nach Anspruch 5, **dadurch gekennzeichnet, dass** die Vorrichtungen (18) zur Bestimmung eines signifikanten Parameters der Blutkonzentration bereitgestellt werden, um den zweiten Wert (HGBA3) des signifikanten Parameters der Blutkonzentration im arteriellen Schlauch (13) im stabilisierten Zustand nach Beginn der ersten Veränderung des Ultrafiltrationsdurchsatzes zu bestimmen.

7. System nach einem der Ansprüche 2 bis 6, **dadurch gekennzeichnet, dass** die Vorrichtungen zum Bewirken einer ersten Veränderung des Ultrafiltrationsdurchsatzes vor dem Schritt des Bewirkens einer ersten Veränderung des Ultrafiltrationsdurchsatzes den Ultrafiltrationsdurchsatz in der Behandlungsapparatur auf im Wesentlichen gleich null regulieren.

8. System nach Anspruch 7, **dadurch gekennzeichnet, dass** die Vorrichtungen zum Bewirken einer ersten Veränderung der vorbestimmten Amplitude ($Q_{UF}$) des Ultrafiltrationsdurchsatzes in der Behandlungsapparatur bereitgestellt werden, um den Ultrafiltrationsdurchsatz zu erhöhen.

9. System nach Anspruch 8, **dadurch gekennzeichnet, dass** die Vorrichtungen zur Berechnung der Rezirkulation (AR) des Bluts im extrakorporalen Kreislauf bereitgestellt werden, um die folgende Gleichung zu lösen:

$$A_R\ \% = \frac{Q_B - Q_{UF}}{Q_B + Q_{UF}\left(\frac{HGBS}{HGBA_3 - HGBS}\right)}\ 100$$

worin:

- $Q_B$ der Durchsatz von Blut im extrakorporalen Kreislauf ist,
- $Q_{UF}$ die vorbestimmte Amplitude der ersten Veränderung des Ultrafiltrationsdurchsatzes ist,
- HGBS der Wert der systemischen Blutkonzentration und gleich dem ersten Wert (HGBA0) des signifikanten Parameters der Blutkonzentration im arteriellen Schlauch (13) ist und
- HGBA3 der zweite Wert des signifikanten Parameters der Blutkonzentration im arteriellen Schlauch (13) ist.

10. System nach einem der Ansprüche 2 bis 6, **dadurch gekennzeichnet, dass** die Vorrichtungen zum Bewirken einer ersten Veränderung der vorbestimmten Amplitude ($Q_{UF}$) des Ultrafiltrationsdurchsatzes in der Behandlungsapparatur bereitgestellt werden, um einen bestehenden Ultrafiltrationsdurchsatz zu verringern, bis dieser im Wesentlichen gleich null wird.

11. System nach Anspruch 10, **dadurch gekennzeichnet, dass** die Vorrichtungen zur Berechnung der Rezirkulation (AR) des Bluts im extrakorporalen Kreislauf bereitgestellt werden, um die folgende Gleichung anzuwenden:

$$A_R\ \% = \frac{Q_B - Q_{UF}}{Q_B + Q_{UF}\left(\frac{HGBS}{HGBA_0 - HGBS}\right)}\ 100$$

worin:

- $Q_B$ der Durchsatz von Blut im extrakorporalen Kreislauf ist,
- $Q_{UF}$ die vorbestimmte Amplitude der ersten Veränderung des Ultrafiltrationsdurchsatzes ist,
- HGBA0 der erste Wert des signifikanten Parameters der Blutkonzentration im arteriellen Schlauch (13) ist,
- HGBS der Wert der systemischen Blutkonzentration und gleich dem zweiten Wert (HGBA3) des signifikanten Parameters der Blutkonzentration im arteriellen Schlauch (13) ist.

**12.** System nach einem der Ansprüche 1 bis 11, **dadurch gekennzeichnet, dass** die Behandlungsapparatur ein Dialysator (12) ist, in dem eine Dialyseflüssigkeit in Zirkulation gebracht werden kann, und dadurch, dass das System Vorrichtungen (15) zum Unterbrechen der Zirkulation der Dialyseflüssigkeit vor dem Schritt der Bestimmung eines ersten Werts des signifikanten Parameters der Konzentration (HGBA0) des Bluts im extrakorporalen Kreislauf umfasst.

**13.** System nach einem der Ansprüche 1 bis 12, **dadurch gekennzeichnet, dass** das System Vorrichtungen (15) zum Wiedereinleiten der Zirkulation von Dialyseflüssigkeit umfasst, wenn die notwendigen Messungen zur Bestimmung der Rezirkulation durchgeführt worden sind.

**14.** System nach einem der Ansprüche 1 bis 13, **dadurch gekennzeichnet, dass** die Vorrichtungen (18) zur Bestimmung eines signifikanten Parameters der Blutkonzentration im arteriellen Schlauch bereitgestellt werden, um das Konzentrationssignal während der gesamten zur Bestimmung der Rezirkulation notwendigen Messung aufzunehmen und zu speichern.

**15.** System nach einem der Ansprüche 1 bis 9, **dadurch gekennzeichnet, dass** die Vorrichtungen zum Bewirken einer ersten Veränderung des Ultrafiltrationsdurchsatzes bereitgestellt werden, um einen Ultrafiltrationsdurchsatz gleich drei Litern pro Stunde für fünf Minuten einzustellen.

**16.** System nach einem der Ansprüche 1 bis 15, **dadurch gekennzeichnet, dass** der signifikante Parameter der Blutkonzentration die Hämoglobinkonzentration des Bluts ist.

Fig.1

Fig.2

Fig. 3

30 — DEFINITION
DE $Q_B$

31 — ACTIVATION
HEMOGLOBINOMETRE

32 — APPAREIL
DE DIALYSE
EN DERIVATION

33 — $Q_{UF} = 0 \, L/H$

34 — DETERMINATION
DE $HGBA_0$ ET HGBS

35 — $Q_{UF} = 3 \, L/H$

DEBUT

FIN

36 — DETERMINATION
DE $HGBA_1$ ET $HGBA_2$

37 — DETERMINATION
DE LA DROITE
D'INTERPOLATION

38 — CORRECTION
DU SIGNAL $C_B$

39 — DETERMINATION
DE $HGBA_3$

40 — CALCUL DE
LA VALEUR DE $A_R$

# FIG. 4

EP 0 886 529 B1

Qu̶F

3ℓ/h

t

**Fig.5**

Cᴇ

t

**Fig.6**

Cᴇ

HGBA₁

HGBA₂

t₁     t₂     t

**Fig.7**